(19) Europäisches Patentamt
European Patent Office
Office européen des brevets

(11) **EP 0 788 787 B1**

(12) **EUROPÄISCHE PATENTSCHRIFT**

(45) Veröffentlichungstag und Bekanntmachung des Hinweises auf die Patenterteilung:
**19.09.2001 Patentblatt 2001/38**

(51) Int Cl.7: **A61K 7/13**

(21) Anmeldenummer: **96117809.2**

(22) Anmeldetag: **07.11.1996**

(54) **Oxidationshaarfärbemittel auf der Basis einer gelförmigen Trägermasse**

Oxidation hair dye composition based upon a gel carrier

Composition de teinture des cheveux par oxydation à base d'un véhicule gélifié

(84) Benannte Vertragsstaaten:
**DE ES FR GB IT**

(30) Priorität: **10.02.1996 DE 19604932**

(43) Veröffentlichungstag der Anmeldung:
**13.08.1997 Patentblatt 1997/33**

(73) Patentinhaber: **Wella Aktiengesellschaft**
**64274 Darmstadt (DE)**

(72) Erfinder:
• **Mager, Herbert, Dr.**
**1723 Marly (CH)**
• **Aeby, Johann**
**1723 Marly (CH)**

(56) Entgegenhaltungen:
**EP-A- 0 258 586          EP-A- 0 642 783**
**WO-A-94/04125          DE-A- 4 005 008**
**FR-A- 2 434 619          FR-A- 2 532 174**

• **CHEMICAL ABSTRACTS, vol. 123, no. 26, 25.Dezember 1995 Columbus, Ohio, US; abstract no. 349858p, Y. NAKAMA ET AL: "Hair-dyeing compositions containing surfactants, fatty acids, and oxidizing agents for mixing at the time of use and the method for mixing the compositions" Seite 681; XP002039203 & JP 07 233 035 A (SHISEIDO) 5.September 1995**

## Beschreibung

Die vorliegende Erfindung betrifft ein Oxidationshaarfärbemittel auf der Basis einer gelförmigen Trägermasse.

Als Farbträgermassen für Haarfarbstoffe werden bei der oxidativen Haarfärbung in der Regel Cremes, Gele oder Flüssigkeiten verwendet.

Zwecks Verbesserung der Haarbenetzung sowie der Ausspülbarkeit des Haafärbemittels werden der Farbträgermasse in der Regel Tenside zugesetzt.

Der Einsatz von gelförmigen Farbträgermassen ist weit verbreitet, wobei insbesondere solche Trägermassen von Interesse sind, die gleichzeitig auch die Eigenschaften eines Haarwaschmittels besitzen. Hierdurch ist eine einfache Anwendung durch Einschäumen der Haare möglich. Das ansonsten übliche Nachwaschen der Haare mit einem Shampoo ist bei diesen Mitteln nicht mehr erforderlich, da eine derartige Tönungswäsche die Funktion der Haarfärbung und Haarwäsche miteinander kombiniert.

Problematisch bei diesen gelförmigen Farbträgermassen ist ihr Gehalt an verdickenden Polymeren und Ölen oder Wachsen (zum Beispiel Fettalkoholen), da durch die Polymeren die Verteilbarkeit und Anschäumbarkeit beeinträchtigt wird und die Öle oder Wachse die Schaumentwicklung vermindern.

Ein weiteres Problem besteht darin, daß der Gehalt an Tensiden aus Kostengründen, aber auch aus Gründen einer besseren Hautverträglichkeit, möglichst gering sein sollte.

CHEMICAL Abstract, vol. 123, no. 26, 25 Dezember 1995 Colombus, Ohio, US; Abstract no. 349858p und Kokai JP-07,233,035 [95,233,035] offenbart Haarfärbemittel, die Tenside, Fettsäuren und Oxidationsmittel enthalten.

Aufgabe der Erfindung war es daher, ein Haarfärbemittel auf der Basis einer gelförmigen Trägermasse zur Verfügung zu stellen, welches selbstverdickend ist, das heißt, bei dem zur Verdickung keine Polymere zugesetzt werden müssen, eine sehr gute Schaumwirkung und Ausspülbarkeit aufweist und kostengünstig hergestellt werden kann.

Es wurde nunmehr gefunden, daß die vorstehende Aufgabe durch ein Mittel zum Färben der Haare, welches eine Kombination aus bestimmten Rohstoffen enthält, in hervorragender Weise gelöst werden kann.

Gegenstand der vorliegenden Erfindung ist daher ein Mittel zur oxidativen Färbung von Haaren auf der Basis einer gelförmigen Trägermasse und eines darin gelösten Farbstoffgemisches, welches dadurch gekennzeichnet ist, daß es

(A) Ölsäure und/oder Oleate,

(B) Natriumlaurylethersulfat (mit 2 bis 4 Mol Ethylenoxid im Molekül) und/oder Natriummyristylethersulfat (mit 2 bis 4 Mol Ethylenoxid im Molekül) und

(C) mindestens ein anorganisches Salz enthält,

einen pH-Wert von 7 bis 12,5 aufweist und frei von Ölen und Wachsen ist.

In dem neuen Haarfärbemittel wird die Ölsäure und/oder das Oleat der Komponente (A) vorzugsweise in einer Gesamtmenge von 0,5 bis 8 Gewichtsprozent eingesetzt.

Die bevorzugte Einsatzmenge für das Natriumlaurylethersulfat (mit 2 bis 4 Mol Ethylenoxid im Molekül) und/oder das Natriummyristylethersulfat (mit 2 bis 4 Mol Ethylenoxid im Molekül) der Komponente (B) beträgt insgesamt 2 bis 10 Gewichtsprozent.

Die Einsatzmenge für das anorganische Salz der Komponente (C) beträgt vorzugsweise 0,05 bis 5 Gewichtsprozent, wobei als anorganisches Salz insbesondere Natriumchlorid eingesetzt wird.

Darüber hinaus kann die Trägermasse noch weitere für solche Mittel übliche Zusätze, beispielsweise Antioxidantien, wie zum Beispiel Ascorbinsäure, Thioglykolsäure oder Natriumsulfit, Parfümöle, Pflegestoffe, wie zum Beispiel Lanolinderivate, Cholesterin, Betain und Pantothensäure, Komplexbildner, Netzmittel und Emulgatoren enthalten.

Die erwähnten Zusatzstoffe werden in den für solche Zwecke üblichen Mengen verwendet, zum Beispiel die Pflegestoffe in einer Menge von etwa 0,1 bis 5 Gewichtsprozent, die Antioxidantien und Parfümöle in einer Menge von jeweils bis zu etwa 1 Gewichtsprozent und die Komplexbildner in einer Menge von etwa 0,1 bis 5 Gewichtsprozent.

Obwohl der Zusatz von Netzmitteln und Emulgatoren möglich ist, enthält das erfindungsgemäße Haarfärbemittel vorzugsweise außer den Tensiden der Komponente (A) und (B) keine weiteren Netzmittel und Emulgatoren.

Die Trägermasse enthält zur pH-Wert Einstellung aliphatische Amine, wobei die Verwendung von 2-Amino-2-methyl-1-propanol und Monoethanolamin besonders bevorzugt ist.

Das in dem neuen gelförmigen Haarfärbemittel enthaltene Farbstoffgemisch besteht aus mindestens einer Entwicklersubstanz und mindestens einer Kupplersubstanz. Gegebenenfalls können zusätzlich mit sich selbst kuppelnde Farbvorstufen und direkt auf das Haar aufziehende Farbstoffe enthalten sein.

Die Entwickler- und Kupplersubstanzen werden in dem Haarfärbemittel entweder als solche oder in Form ihrer physiologisch unbedenklichen Salze mit anorganischen oder organischen Säuren, zum Beispiel als Hydrochlorid, Sulfat, Phosphat oder Acetat, oder - sofern sie aromatische OH-Gruppen besitzen - in Form der Salze mit Basen, beispielsweise als Alkaliphenolate, eingesetzt.

Von den bekannten Entwicklersubstanzen kommen als Bestandteil des neuen Haarfärbemittels vorzugsweise 1,4-Diaminobenzol, 2,5-Diaminotoluol, 2,5-Diaminophenylethanol, 4-Amino-3-methylphenol, 4-Amino-2-aminomethylphenol und Tetraaminopyrimidin in Betracht.

Als geeignete Kupplersubstanzen können insbesondere Resorcin, 4-Chlorresorcin, 2-Methylresorcin, 5-Amino-2-methylphenol, 3-Aminophenol, 3,4-Methylendioxyphenol, 3,4-Methylendioxyanilin, 4-(2'-Hydroxyethylamino)-1,2-methylendioxybenzol, 4-Hydroxyindol, 4-(2'-Hydroxyethylamino)-2-amino-anisol, 1,3-Diaminobenzol, 3,5-Diamino-2,6-dimethoxypyridin und 1-Naphthol genannt werden.

Die Gesamtmenge der in dem hier beschriebenen Haarfärbemittel enthaltenen Entwicklersubstanz-Kupplersubstanz-Kombination soll etwa 0,1 bis 8 Gewichtsprozent, vorzugsweise etwa 0,5 bis 4 Gewichtsprozent, betragen.

Die Entwicklersubstanzen und Kupplersubstanzen werden im allgemeinen in äquimolaren Mengen eingesetzt. Es ist jedoch nicht nachteilig, wenn eine dieser beiden Verbindungen in einem gewissen Überschuß oder Unterschuß eingesetzt wird.

Ferner können auch weitere Farbstoffe, wie zum Beispiel 6-Amino-2-methyl-phenol, 2-Amino-5-methyl-phenol sowie ferner direktziehende Farbstoffe, wie zum Beispiel Triphenylmethanfarbstoffe, wie beispielsweise C. I. Basic Violet 14 (C. I. 42510) und C. I. Basic Violet 2 (C. I. 42520), aromatische Nitrofarbstoffe, wie zum Beispiel 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)-nitrobenzol und 2-Methylamino-5-bis-(2'-hydroxyethylamino)-nitrobenzol, Azofarbstoffe, wie zum Beispiel C. I. Acid Brown 4 (C. I. 14805) und Dispersionsfarbstoffe, wie zum Beispiel 1,4-Diaminoanthrachinon und 1,4,5,8-Tetraaminoanthrachinon, zugesetzt werden.

Das Haarfärbemittel kann diese Farbstoffe in einer Gesamtmenge von etwa 0,1 bis 4 Gewichtsprozent enthalten, wobei die Gesamtmenge des Farbstoffgemisches aus diesen Farbstoffen und der Entwicklersubstanz-Kupplersubstanz-Kombination etwa 0,1 bis 8 Gewichtsprozent, vorzugsweise 0,5 bis 5 Gewichtsprozent, beträgt.

Der pH-Wert des erfindungsgemäßen Haarfärbemittels beträgt 7 bis 12,5, wobei ein pH-Wert von 8 bis 11,5 bevorzugt ist.

Das erfindungsgemäße Oxidationshaarfärbemittel stellt ein Gemisch aus der gelförmigen Trägermasse und dem Farbstoffgemisch dar und besitzt eine Viskosität von 100 bis 5000 mPa·s.

Bei der Anwendung vermischt man das Haarfärbemittel unmittelbar vor dem Gebrauch in einem Gewichtsverhältnis von etwa 1:1 bis 8:1, vorzugsweise von etwa 5:1 bis 3:1, mit einem Oxidationsmittel und trägt eine für die Färbebehandlung ausreichende Menge, je nach Haarfülle, im allgemeinen etwa 60 bis 200 g, dieses Gemisches auf das Haar auf. Als Oxidationsmittel zur Entwicklung der Haarfärbung kommt insbesondere Wasserstoffperoxid, vorzugsweise als 1- bis 12prozentige wäßrige Lösung oder Emulsion, oder aber in Form seiner Additionsverbindungen an Harnstoff, Melamin oder Natriumborat, in Betracht.

Das so erhaltene gebrauchsfertige Haarfärbemittel besitzt eine pH-Wert von 7 bis 12,5 und weist eine Viskosität von 30 bis 800 mPa·s auf.

Man läßt das Gemisch bei 15 bis 50 Grad Celsius etwa 10 bis 45 Minuten lang, vorzugsweise 30 Minuten lang, auf das Haar einwirken, spült sodann das Färbegemisch mit Wasser aus und trocknet das Haar. Gegebenenfalls wird im Anschluß an diese Spülung mit einer schwachen physiologisch verträglichen organischen Säure, wie zum Beispiel Zitronensäure oder Weinsäure nachgespült.

Das durch die erfindungsgemäße gelförmige Trägermasse gekennzeichnete neue Haarfärbemittel ist selbstverdickend und benötigt daher keinen Zusatz von verdickenden Polymeren. Weiterhin besitzt das erfindungsgemäße Haarfärbemittel ein hervorragendes Schaumvermögen und gute Shampooeigenschaften.

Die nachfolgenden Beispiele sollen den Gegenstand der Erfindung näher erläutern, ohne diesen hierauf zu beschränken.

**Beispiele**

**Beispiel 1:** Gelförmiges Oxidationshaarfärbemittel

| | |
|---|---|
| 4,40 g | Ölsäure |
| 14,00 g | Natriumlaurylalkoholdiglykolethersulfat (28prozentige wäßrige Lösung) |
| 2,98 g | Monoethanolamin |
| 0,80 g | Natriumchlorid |
| 0,10 g | Natriumsulfit |

(fortgesetzt)

| | |
|---|---|
| 0,74 g | 3-Amino-6-methylphenol |
| 0,70 g | 2,5-Diaminotoluol-Sulfat |
| 0,28 g | 4-Amino-3-methylphenol |
| 0,07 g | 2-Chlor-6-ethylamino-4-nitrophenol |
| 0,30 g | Ethylendiaminotetraessigsäure-Dinatriumsalz |
| 0,30 g | Parfümöl |
| 75,33 g | Wasser |
| 100,00 g | |

Das vorgenannte Mittel besitzt einen pH-Wert von 10,2 und eine Viskosität von 850 mPa·s.

50 g des vorstehend beschriebenen gelförmigen Oxidationshaarfärbemittels werden mit 10 g einer 6prozentigen wäßrigen Wasserstoffperoxidlösung (pH-Wert gleich 2,3 bis 4,5) zu einem gebrauchsfertigen Haarfärbemittel mit pH = 10,0 und einer Viskosität von 205 mPa·s vermischt und auf blondes Naturhaar aufgetragen. Nach einer Einwirkungszeit von 30 Minuten wird das Haar mit lauwarmem Wasser gespült und sodann getrocknet. Das so behandelte Haar weist eine intensive rote bis purpurfarbene Färbung auf.

**Beispiel 2:** Vergleichsversuche

**Beispie 2a:** Oxidationshaarfärbemittel (nicht-erfindungsgemäß/ohne Ölsäure)

| | |
|---|---|
| 14,00 g | Natriumlaurylalkoholdiglykolethersulfat (28prozentige wäßrige Lösung) |
| 2,00 g | Monoethanolamin |
| 0,80 g | Natriumchlorid |
| 0,10 g | Natriumsulfit |
| 0,74 g | 3-Amino-6-methylphenol |
| 0,70 g | 2,5-Diaminotoluol-Sulfat |
| 0,28 g | 4-Amino-3-methylphenol |
| 0,07 g | 2-Chlor-6-ethylamino-4-nitrophenol |
| 0,30 g | Ethylendiaminotetraessigsäure-Dinatriumsalz |
| 0,30 g | Parfümöl |
| 80,71 g | Wasser |
| 100,00 g | |

Das vorstehend beschriebene Oxidationshaarfärbemittel gemäß Beispiel 2a (pH = 10,3, Viskosität = 12 mPa·s) wird in der in Beispiel 1 beschriebenen Weise angewandt. Die gebrauchsfertige Färbezubereitung besitzt einen pH-Wert von 10,2 und eine Viskosität von 10 mPa·s. Es wird der gleiche Farbton wie bei Beispiel 1 erhalten.

Das nicht-erfindungsgemäße Oxidationshaarfärbemittel ist jedoch zur Haarfärbung nicht geeignet, da es aufgrund seiner geringen Viskosität während der Einwirkungszeit vom Haar abläuft.

**Beispiel 2b:** Abhängigkeit der Viskosität von der Salzkonzentration

Wie das Diagramm der Anlage 1 zeigt, ist eine ausreichende Viskosität des gebrauchsfertigen Haarfärbemittels nur bei Verwendung der erfindungsgemäßen Kombination aus den Komponenten (A), (B) und (C) erzielbar, während beim Weglassen einer dieser Komponenten keine ausreichende Verdickung mehr erzielt werden kann.

Weiterhin zeigt Anlage 1, daß bei dem erfindungsgemäßen Haarfärbemittel bereits durch eine geringfügige Erhöhung der Salzkonzentration eine deutliche Viskositätssteigerung erzielt wird.

Die in dieser Anmeldung verwendeten Prozentangaben stellen, soweit nicht anders vermerkt, Gewichtsprozente dar. Die Ermittlung der Viskositätswerte erfolgte mit einem Rotationsviskosimeter der Firma Haake, Typ VT 501 Meßeinrichtung MV (DIN 53 018), Geschwindigkeitsgefälle D = 12,9 s$^{-1}$, bei einer Temperatur von 25 Grad Celsius.

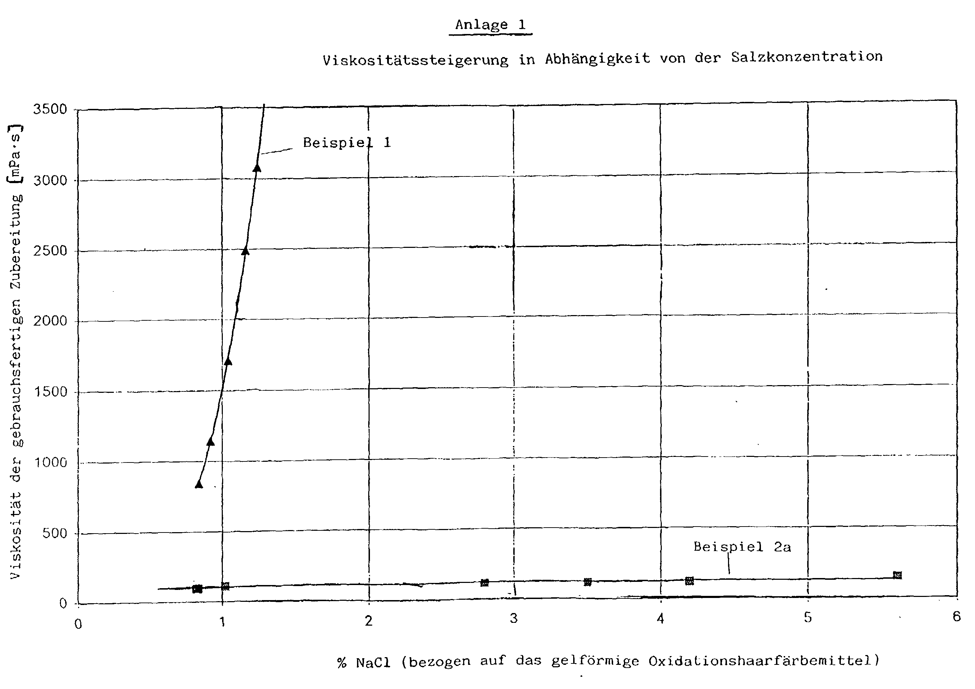

**Patentansprüche**

1. Mittel zur oxidativen Färbung von Haaren auf der Basis einer gelförmigen Trägermasse und eines darin gelösten Farbstoffgemisches, **dadurch gekennzeichnet, daß** es

   (A) Ölsäure und/oder Oleate,

   (B) Natriumlaurylethersulfat (mit 2 bis 4 Mol Ethylenoxid im Molekül) und/oder Natriummyristylethersulfat (mit 2 bis 4 Mol Ethylenoxid im Molekül), und

   (C) mindestens ein anorganisches Salz enthält,
       einen pH-Wert von 7 bis 12,5 aufweist und frei von Ölen und Wachsen ist.

2. Mittel nach Anspruch 1, **dadurch gekennzeichnet, daß** die Komponente (A) in einer Menge von 0,5 bis 8 Gewichtsprozent enthalten ist.

3. Mittel nach Anspruch 1 oder 2, **dadurch gekennzeichnet, daß** die Komponente (B) in einer Menge von 2 bis 10 Gewichtsprozent enthalten ist.

4. Mittel nach einem der Ansprüche 1 bis 3, **dadurch gekennzeichnet, daß** die Komponente (C) in einer Menge von 0,05 bis 5 Gewichtsprozent enthalten ist.

5. Mittel nach einem der Ansprüche 1 bis 4, **dadurch gekennzeichnet, daß** als anorganisches Salz der Komponente (C) Natriumchlorid eingesetzt wird.

6. Mittel nach einem der Ansprüche 1 bis 5, **dadurch gekennzeichnet, daß** das in der Trägermasse gelöste Farbstoffgemisch mindestens eine Entwicklersubstanz aus der Gruppe bestehend aus 1,4-Diaminobenzol, 2,5-Diaminotoluol, 2,5-Diaminophenylethanol, 4-Amino-3-methylphenol, 4-Amino-2-aminomethylphenol und Tetraaminopyrimidin enthält.

7. Mittel nach einem der Ansprüche 1 bis 6, **dadurch gekennzeichnet, daß** das in der Trägermasse gelöste Farbstoffgemisch mindestens eine Kupplersubstanz aus der Gruppe bestehend aus Resorcin, 4-Chlorresorcin, 5-Amino-2-methylphenol, 3-Aminophenol, 2-Methylresorcin, 3,4-Methylendioxyphenol, 3,4-Methylendioxyanilin, 4-(2'-Hydroxyethylamino)-1,2-methylendioxybenzol, 4-Hydroxyindol, 4-(2'-Hydroxyethylamino)-2-amino-anisol, 1,3-Diaminobenzol, 3,5-Diamino-2,6-dimethoxypyridin und 1-Naphthol enthält.

8. Mittel nach einem der Ansprüche 1 bis 7, **dadurch gekennzeichnet, daß** die Gesamtmenge der in dem Haarfärbemittel enthaltenen Entwicklersubstanz-Kupplersubstanz-Kombination 0,1 bis 8 Gewichtsprozent beträgt.

9. Mittel nach einem der Ansprüche 1 bis 8, **dadurch gekennzeichnet, daß** die Einstellung des pH-Wertes mit aliphatischen Aminen erfolgt.

10. Mittel nach Anspruch 9, **dadurch gekennzeichnet, daß** als aliphatisches Amin Monoethanolamin oder 2-Amino-2-methyl-1-propanol eingesetzt wird.

11. Mittel nach einem der Ansprüche 1 bis 10, **dadurch gekennzeichnet, daß** das in der Trägermasse gelöste Farbstoffgemisch mindestens einen Farbstoff aus der Gruppe bestehend aus 6-Amino-2-methyl-phenol, 2-Amino-5-methyl-phenol, C. I. Basic Violet 14, C. I. Basic Violet 2, 2-Nitro-1,4-diaminobenzol, 2-Amino-4-nitrophenol, 2-Amino-5-nitrophenol, 2-Amino-4,6-dinitrophenol, 2-Amino-5-(2'-hydroxyehtylamino)-nitrobenzol, 2-Methylamino-5-bis-(2'-hydroxyethylamino)-nitrobenzol, C. I. Acid Brown 4, l,4-Diaminoanthrachinon und 1,4,5,8-Tetraaminoanthrachinon enthält.

12. Verfahren zum oxidativen Färben von Haaren, **dadurch gekennzeichnet, daß** man ein Haarfärbemittel nach einem der Ansprüche 1 bis 10 unmittelbar vor dem Gebrauch mit einem Oxidationsmittel im Verhältnis 1:1 bis 8:1 vermischt, anschließend eine für die Haarfärbung ausreichende Menge dieses Gemisches auf das Haar aufträgt und bei 15 bis 50 Grad Celsius 10 bis 45 Minuten lang auf das Haar einwirken läßt, das Haar mit Wasser spült und sodann trocknet.

13. Verfahren nach Anspruch 12, **dadurch gekennzeichnet, daß** als Oxidationsmittel eine 1 bis 12 Gewichtsprozent Wasserstoffperoxid enthaltende wäßrige Wasserstoffperoxidlösung oder -emulsion eingesetzt wird.

**Claims**

1. Agent for oxidative colouring of hair on the basis of a carrier mass in the form of gel and a dye mixture dissolved therein, **characterised in that** it contains

   (A) oleic acid and/or oleate;

   (B) sodium lauryl ether sulphate (with 2 to 4 mol of ethylene oxide in the molecule) and/or sodium myristyl ether sulphate (with 2 to 4 mol ethleneoxide in the molecule); and

   (C) contains at least one inorganic salt;
   has a pH value of 7 to 12.5 and does not contain oil or waxes.

2. Agent according to Claim 1, **characterised in that** the component (A) is contained in an amount of from 0.5 to 8 weight per cent.

3. Agent according to Claim 1 or 2, **characterised in that** the component (B) is contained in an amount of 2 to 10 weight per cent.

4. Agent according to one of Claims 1 to 3, **characterised in that** the component (C) is contained in an amount of from 0.05 to 5 weight per cent.

5. Agent according to one of Claims 1 to 4, **characterised in that** component (C), sodium chloride, is used as the inorganic salt.

6. Agent according to one of Claims 1 to 5, **characterised in that** the dye mixture which is dissolved in the carrier mass contains at least one developer substance from the group consisting of 1,4-diaminobenzene, 2,5-diaminotoluene, 2.5-diaminophenylethanol, 4-amino-3-methylphenol, 4-amino-2-aminomethylphenol and tetraaminopyrimidin.

7. Agent according to one of Claims 1 to 6, **characterised in that** the dye mixture which is dissolved in the carrier mass contains at least one coupler substance from the group consisting of resorcinol, 4-chlororesorcinol, 5-amino-2-methylphenol, 3-aminophenol, 2-methylresorcinol, 3,4-methylendioxyphenol, 3,4-methylenedioxyaniline, 4-(2'-hydroxyethylamino)-1,2-methylenedioxybenzene, 4-hydroxyindole, 4-(2'-hydroxyethylamino)-2-amino-anisole, 1,3-diaminobenzene, 3,5-diamino-2,6-dimethoxypyridine and 1-naphthene.

8. Agent according to any one of Claims 1 to 7, **characterised in that** the total amount of the developer substance/coupler substance combination which is contained in the hair-colouring agent is 0.1 to 8 per cent by weight.

9. Agent according to any one of Claims 1 to 8, **characterised in that** the pH-values are adjusted with aliphatic amines.

10. Agent according to Claim 9, **characterised in that** monoethanolamine or 2-amino-2-methyl-1-propanol is used as the aliphatic amine.

11. Agent according to one of Claims 1 to 10, **characterised in that** the dye substance which is dissolved in the carrier mass has at least one dye from the group consisting of 6-amino-2-methylphenol, 2-amino-5-methyl-phenol, C. I. Basic Violet 14, C. I. Basic Violet 2, 2-nitro-1,4-diaminobenzene, 2-amino-4-nitrophenol, 2-amino-5-nitrophenol, 2-amino-4,6-dinitrophenol, 2-amino-5-(2'-hydroxyethylamino)-nitrobenzene, 2-methylamino-5-bis-(2'-hydroxyethylamino)-nitrobenzene, C. I. Acid Brown 4, 1,4-diaminoanthraquinone and 1,4,5,8-Tetraaminoanthraquinone.

12. Process for the oxidative colouring of hair, **characterised in that** a hair-colouring agent according to one of Claims 1 to 10 is mixed, immediately before application, with an oxidation agent in the ratio of from 1:1 to 8:1, applied to the hair in an amount which is sufficient for the colouring of the hair and allowed to act on the hair for 10 to 45 minutes at 15 to 50 degrees celsius, and the then hair is rinsed with water and then dried.

13. Process according to Claim 12, **characterised in that** an aqueous hydrogen peroxide solution or emulsion which contains 1 to 12 per cent by weight of hydrogen peroxide is used as the oxidation agent.

**Revendications**

1. Agent de teinture oxydative des cheveux, à base d'une masse-support se présentant sous forme de gel et d'un mélange de teinture qui y est dissous, **caractérisé en ce qu'**il contient

   (A) de l'acide oléique et/ou des oléates,
   (B) du lauryléthersulfate de sodium (comportant de 2 à 4 moles d'oxyde d'éthylène dans la molécule) et/ou du myristyléthersulfate de sodium (comportant de 2 à 4 moles d'oxyde d'éthylène dans la molécule), et
   (C) au moins un sel inorganique,

   il présente un pH d'une valeur comprise dans la plage allant de 7 à 12,5 et est exempt d'huiles et de paraffine.

2. Agent selon la revendication 1, **caractérisé en ce que** le composant (A) est contenu en une quantité comprise dans la plage allant de 0,5 à 8 pour-cent en poids.

3. Agent selon la revendication 1 ou 2, **caractérisé en ce que** le composant (B) est contenu en une quantité comprise dans la plage allant de 2 à 10 pour-cent en poids.

4. Agent selon l'une des revendications 1 à 3, **caractérisé en ce que** le composant (C) est contenu en une quantité comprise dans la plage allant de 0,05 à 5 pour-cent en poids.

5. Agent selon l'une des revendications 1 à 4, **caractérisé en ce qu'**est utilisé comme sel inorganique du composant (C) du chlorure de sodium.

6. Agent selon l'une des revendications 1 à 5, **caractérisé en ce que** le mélange de teinture dissous dans la masse-support contient au moins un développeur issu du groupe constitué du 1,4-diaminobenzène, 2,5-diaminotoluène, 2,5-diaminophényléthanol, 4-amino-3-méthylphénol, 4-amino-2-aminométhylphénol et de la tétraaminopyrimidine.

7. Agent selon l'une des revendications 1 à 6, **caractérisé en ce que** le mélange de teinture dissous dans la masse-support contient au moins un copulateur issu du groupe constitué de la résorcine, de la 4-chlororésorcine, du 5-amino-2-méthylphénol, du 3-aminophénol, de la 2-méthylrésorcine, du 3,4-méthylènedioxyphénol, de la 3,4-méthylènedioxyaniline, du 4-(2'-hydroxyéthylamino)-1,2-méthylènedioxybenzène, 4-hydroxyindol, 4-(2'-hydroxyéthyl-amino)-2-amino-anisol, 1,3-diaminobenzène, de la 3,5-diamino-2,6-diméthoxypyridine et du 1-naphtol.

8. Agent selon l'une des revendications 1 à 7, **caractérisé en ce que** la quantité totale de combinaison développeur-copulateur contenue dans l'agent de teinture capillaire est de 0,1 à 8 pour-cent en poids.

9. Agent selon l'une des revendications 1 à 8, **caractérisé en ce que** le réglage de la valeur du pH s'effectue avec des amines aliphatiques.

10. Agent selon la revendication 9, **caractérisé en ce qu'**on utilise comme amine aliphatique de la monoéthanolamine ou du 2-amino-2-méthyl-1-propanoL

11. Agent selon l'une des revendications 1 à 10, **caractérisé en ce que** le mélange de teinture dissous dans la masse-support contient au moins une teinture issue du groupe constitué de 6-amino-2-méthyl-phénol, 2-amino-5-méthyl-phénol, C.I. Basic Violet 14, C.L Basic Violet 2, 2-nitro-1,4-diaminobenzène, 2-amine-4-nitrophénol, 2-amino-5-nitrophénol, 2-amino-4,6-dinitrophénol, 2-amino-5-(2'-hydroxyéthylamino)-nitrobenzène, 2-méthylamino-5-bis-(2'-hydroxyéthylamino)-nitrobenzène, C.L Acid Brown 4, 1,4-diaminoanthraquinone et 1,4,5,8-tétraaminoanthraquinone.

12. Procédé de teinture oxydative des cheveux, **caractérisé en ce qu'**on mélange un agent de teinture capillaire selon l'une des revendications 1 à 10, directement avant utilisation, avec un agent d'oxydation suivant un rapport 1:1 à 8:1, puis on applique une quantité, suffisante pour la teinture capillaire, de ce mélange sur les cheveux et on laisse agir sur les cheveux à une température de 15 à 50 degrés Celsius pendant 10 à 45 minutes, on rince les cheveux à l'eau et, ensuite, on sèche.

13. Procédé selon la revendication 12, **caractérisé en ce qu'**on utilise comme agent d'oxydation une solution ou une émulsion aqueuse de peroxyde d'hydrogène contenant de 1 à 12 pour-cent en poids de peroxyde d'hydrogène.

Viskositätssteigerung in Abhängigkeit von der Salzkonzentration

% NaCl (bezogen auf das gelförmige Oxidationshaarfärbemittel)